# EUROPEAN PATENT APPLICATION

(11) **EP 1 811 039 A1**
(43) Date of publication of application: **25.07.2007**
(21) Application number: 05805401.6
(22) Date of filing: 07.11.2005
(51) Int. Cl.: C12Q 1/02, C07K 16/00, G01N 33/543

(54) **METHOD OF EFFICIENTLY SCREENING PROTEIN CAPABLE OF SPECIFIC BINDING TO LIGAND**

(30) Priority: 08.11.2004 JP 2004324217
(71) Applicant: Riken, Wako-shi, Saitama 351-0198 (JP)
(72) Inventor: OHTA, Kunihiro, Wako-shi, Saitama 3510198 (JP); SEO, Hidetaka, Tokyo 1710031 (JP); SHIBATA, Takehiko, Wako-shi, Saitama 3510198 (JP)
(74) Representative: O'Neill, Michelle
(86) International application number: PCT/JP2005/020346
(87) International publication number: WO 2006/049273

(57) **Abstract**

The present invention relates to a method of selecting a protein specifically binding to a specific ligand.

A method of selecting from a diversifying library a protein specifically binding to a ligand of interest, which comprises: a step of allowing proteins existing in the above library to come into contact with the ligand of interest, so as to select proteins binding to the ligand of interest; a step of allowing the obtained proteins to come into contact with a control ligand, so as to determine the presence or absence of the binding ability of the above proteins to the control ligand; and a step of selecting proteins that are determined not to have the binding ability to the control ligand.

## Description

### TECHNICAL FIELD

The present invention relates to a method of selecting a protein specifically binding to a specific ligand.

### BACKGROUND ART

*In vivo* factors, such as a protein, nucleic acid, or lipid, play various roles in a living body. Many of the factors exhibit their functions by themselves, like enzymes. It has been known that such *in vivo* factors transmit information via the interaction between them, so as to induce or regulate various types of *in vivo* reactions. Accordingly, precise clarification of such an interaction between *in vivo* factors is important for understanding such *in vivo* reactions. Moreover, it is considered that accumulation of knowledge in such a reaction mechanism is extremely useful as a key for the development of a treatment method or a therapeutic agent for certain diseases and the like.

As a method of studying the interaction between *in vivo* factors, and in particular, a protein (for example, an antibody, a receptor, etc.) and a ligand specific therefor (for example, a protein, a low molecular weight compound, a lipid, a sugar chain, etc.), there is a method of selecting a protein specifically binding to a specific ligand from a diversifying library such as a phage display. In such a method, when a protein specifically binding to a specific ligand is identified, the specificity or affinity of the protein to the ligand is measured by ELISA (Enzyme-Linked ImmunoSorbent Assay) or RIA, so as to select a molecule specifically binding to the ligand of interest, in many cases (Non-Patent Publications 1 and 2).
Non-Patent Publication 1: Gram et al., Proc. Natl. Acad. Sci., 89: 3576-3580, 1992
Non-Patent Publication 2: Cumbers et al., Nat. Biotechnol., 20: 1129-1134, 2002

### DISCLOSURE OF THE INVENTION

### Problems to be Solved by the Invention

It has been confirmed that many *in vivo* factors generated from a diversifying library, such as a protein, are problematic in that such factors exhibit non-specific binding ability to factors other than a ligand of interest. Thus, it has been revealed that, if a first screening is carried out using only such binding ability to the ligand of interest as an indicator, a majority of factors become non specific binding factors, and thereby it becomes extremely difficult to obtain necessary factors that exhibit specific binding ability.

Under the aforementioned circumstances, the present inventors have conducted intensive studies directed towards achieving a method of selecting a protein exhibiting specific binding ability to a ligand of interest from a diversifying library. As a result, the inventors have discovered a method of easily and efficiently identifying a protein that specifically binds to the ligand of interest.

The present invention relates to a method of selecting a protein that specifically binds to a ligand of interest from a diversifying library.

### Means for Solving the Problems

With regard to the screening of a protein using affinity to a ligand as an indicator, the present inventors have completed a method of easily and efficiently identifying a desired protein, which comprises simultaneously measuring not only affinity to a ligand of interest, but also affinity to a control ligand, making a comparison between the two types of affinities, so as to eliminate proteins that non-specifically bind to the ligand of interest, thereby easily and efficiently identifying such a desired protein.

That is to say, the present invention relates to the following (1) to (11):
(1) The invention in the first embodiment of the present invention relates to "a method of selecting from a diversifying library at least one type of protein specifically binding to a ligand of interest, which comprises:
   (a) a step of allowing various types of proteins existing in the above-described library to come into contact with the ligand of interest, incubating a mixture consisting of the above-described group of proteins and the ligand of interest, and recovering a complex consisting of at least one type of protein and the ligand of interest;
   (b) a step of allowing at least several types of proteins selected in step (a) to come into contact with the ligand of interest, incubating a mixture consisting of the above-described proteins and the ligand of interest, and confirming the binding ability of the above-described proteins to the ligand of interest;
   (c) a step of allowing at least several types of proteins selected in step (a) to come into contact with one or two types of specific control ligand(s), incubating a mixture consisting of the above-described proteins and the control ligand(s), and determining whether or not the above-described proteins have bound to the control ligand(s); and
   (d) a step of selecting proteins, whose binding ability to the ligand of interest has been confirmed in step (b), and which have not bound to the control ligand(s) in step (c)."
(2) The invention in the second embodiment of the present invention relates to "the method according to (1) above, which is characterized in that the proteins that are allowed to come into contact with the above-described ligand of interest in the above step (a) are present on the surface of a host."
(3) The invention in the third embodiment of the present invention relates to "the method according to (1) or (2) above, which is characterized in that the above-described ligand of interest and/or control ligand bind to carriers."
(4) The invention in the fourth embodiment of the present invention relates to "the method according to (3) above, which is characterized in that the above-described carriers are magnetic beads."
(5) The invention in the fifth embodiment of the present invention relates to "the method according to any one of (1) to (4) above, which is characterized in that determination of the presence or absence of binding ability in the above step (b) is carried out by a method selected from the group consisting of an ELISA method, an RIA method, a surface plasmon resonance (SPR) method, a blotting method, and a method using ligand beads."
(6) The invention in the sixth embodiment of the present invention relates to "the method according to (5) above, which is characterized in that the above-described ligand of interest and/or control ligand(s) have been labeled."
(7) The invention in the seventh embodiment of the present invention relates to "the method according to any one of (1) to (6) above, which is characterized in that the above-described diversifying library is a protein expression library."
(8) The invention in the eighth embodiment of the present invention relates to "the method according to (7) above, which is characterized in that the above-described protein expression library is an antibody expression library."
(9) The invention in the ninth embodiment of the present invention relates to "the method according to (8) above, which is characterized in that the above-described antibody expression library is a library that is constituted with cells that present antibodies on the surfaces thereof."
(10) The invention in the tenth embodiment of the present invention relates to "the method according to (9) above, which is characterized in that the above-described cells are DT40 cells."
(11) The invention in the eleventh embodiment of the present invention relates to "a protein, which is selected by the method according to any one of (1) to (10) above."

### Advantages of the Invention

Using the method of the present invention, a protein specifically binding to a ligand of interest can be rapidly and efficiently selected from a diversifying library.

### BRIEF DESCRIPTION OF THE DRAWINGS

Figure 1 shows the results obtained by selecting cells generating antibodies having specific binding ability to streptavidin, from a diversifying library of chicken DT40 cells that present various antibody molecules on the surfaces thereof, using streptavidin magnetic beads, and then performing ELISA on the culture supernatant of the cells. The 22^{nd} clone (arrow) is SD-10.
Figure 2 shows the results obtained by performing ELISA not using ovalbumin but using egg-white lysozyme as a target antigen, when an anti-rabbit IgG antibody is selected.

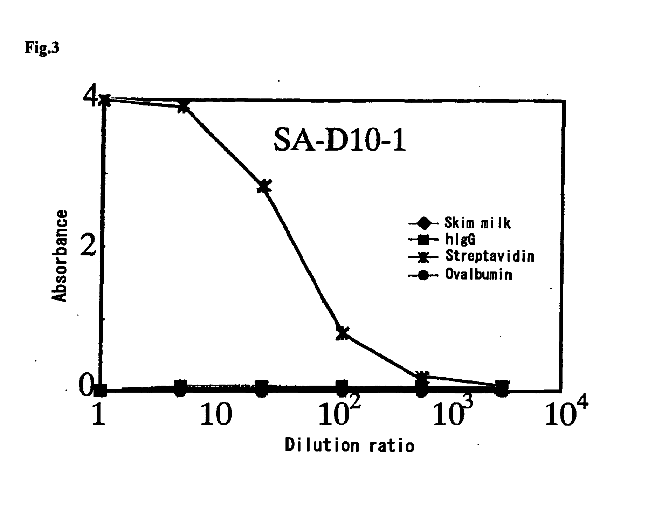

Figure 3 shows the results obtained by performing ELISA to make detailed studies regarding the specificity of the antibody, which has been finally selected in Figure 1, to various ligands (streptavidin, ovalbumin, hIgG (human IgG), and skim milk).

### BEST MODE FOR CARRYING OUT THE INVENTION

### 1. Diversifying library

The term "diversifying library" is used in the present invention to mean a library capable of presenting various types of proteins. The method of the present invention can be applied to any type of library, as long as such a library is able to present various types of proteins. Thus, the type of the library is not limited. Examples of a library that can be used herein include a phage library capable of expressing various types of proteins, and a cell library capable of presenting various types of proteins on the surfaces of cells. A particularly suitable library is a cell library capable of presenting a variety of antibody molecules on the surfaces of cells. An example of the aforementioned "cell library" used herein is a library that is constituted with DT40 cells derived from chick, which is able to present various antibody molecules on the cell surface thereof (WO2004/011644).

The concept of the "protein" of the present invention includes molecules formed by allowing two or more amino acids to bind to one another via a covalent bond (for example, a peptide bond), such as a naturally-derived protein, a mutant thereof, a polypeptide constituting a portion thereof, or an artificially synthesized peptide.

The "protein expression library" of the present invention is included in the aforementioned "diversifying library," and thus the protein expression library has a concept indicating a host population capable of expressing a variety of proteins. The term "host" is used herein to include all hosts that can be conceived of by persons skilled in the art based on common technical knowledge. For example, prokaryocytes, eukaryotes, phages, viruses, etc. are included in such hosts. In addition, a protein that is allowed to express by such a "host" may be allowed to express either in the cells of the above "host," or on the surfaces of the cells. Otherwise, the above "host" may be cultured, so that the protein may also be allowed to express in a medium that is suitable for the expression of the above protein. As such a "protein expression library" that can be applied to the method of the present invention, a commercially available product can also be used.

The "antibody expression library" of the present invention is included in the aforementioned "protein expression library," and thus the antibody expression library has a concept indicating a host population capable of expressing a variety of antibody molecules. The term "host" is used herein to include all hosts that can be conceived of by persons skilled in the art based on common technical knowledge. Examples of such a host include prokaryocytes, eukaryotes, phages, and viruses. Preferred examples include eukaryotes and phages. More preferred examples include animal cells and phages, and particularly preferred examples include DT40 cells and Ramos cells.

### 2. Ligand of interest and control ligand

Any type of a "ligand of interest" can be used in the present invention, as long as such a ligand of interest binds to a protein. Thus, the type of the "ligand of interest" of the present invention is not limited. Examples of such a ligand of interest include all ligands that can be conceived of by persons skilled in the art, such as a protein, a nucleic acid, a lipid, a sugar chain, or a low molecular weight compound. Moreover, any type of a "control ligand" can be used in the present invention, as long as such a control ligand binds to a protein. Thus, the type of the "control ligand" of the present invention is not limited. Examples of such a control ligand include all ligands that can be conceived of by persons skilled in the art, such as a protein, a nucleic acid, a lipid, a sugar chain, or a low molecular weight compound. In terms of the relationship with the "ligand of interest," such a control ligand preferably has low identity at the amino acid sequence level with the "ligand of interest" (approximately 30% or less, more preferably 20% or less, and further more preferably 10% or less), and further the "control ligand" itself does not have non-specific binding activity. The "control ligand" used in the present invention may be either of a single type, or of multiple types. Thus, the number of types is not limited. For example, the control ligand may be of two or less types, and preferably of one type. Moreover, taking into consideration the relationship of the control ligand with the "ligand of interest," several types of proteins, lipids, carbohydrates, etc. contained in skim milk, such as ovalbumin or white-egg lysozyme, may be selected.

Furthermore, such a "ligand of interest" or "control ligand" may also be labeled. For such labeling, any type of labeling method and any type of labeling substance may be applied, as long as such a labeling method and a labeling substance are commonly used in the present technical field. Thus, the type of a labeling method and the type of a labeling substance are not limited. Fluorescent labeling can preferably be used.

### 3. Selection of protein that binds to ligand of interest

In the present invention, a protein binding to a ligand of interest can be selected based on common knowledge in the present technical field. For example, a ligand of interest is allowed to bind to a suitable carrier, and such a bound ligand of interest is then allowed to come into contact with proteins existing in a diversifying library. Thereafter, a mixture consisting of the ligand of interest and the protein is incubated under appropriate conditions, and the generated complex of the carrier-the ligand of interest-the protein is then recovered by centrifugation or the like, so as to select the protein binding to the ligand of interest. When such a protein binding to a ligand of interest is obtained, if a host that expresses the above protein is not a single clone, the single clone can be obtained by the limiting dilution method or subculture. Several proteins as obtained above bind to a ligand of interest via different affinity.

The type of a "carrier" used herein is not limited. Appropriate carriers such as sepharose beads, agarose beads, glass substrates, or magnetic beads, can be used. Magnetic beads are particularly preferable.

Conditions for allowing a ligand of interest to bind to a protein can be determined by persons skilled in the art, depending on the type of the ligand of interest. Such conditions are not particularly limited. When a protein to be selected is an antibody molecule for example, a mixture consisting of the ligand of interest and the protein may be incubated at a temperature between approximately 4°C and 37°C for approximately 15 minutes to 24 hours.

As a method of confirming the binding ability of the thus obtained protein to the ligand of interest, a method that is publicly known in the present technical field can be applied. Examples of such an applicable method include an ELISA method, an RIA method, a surface plasmon resonance (SPR) method, a blotting method, and a method using ligand beads. The ligand of interest used herein may be labeled. When a substance that specifically binds to a protein specifically binding to the ligand of interest (for example, a protein such as an antibody, a nucleic acid, a lipid, etc.) can be obtained, the binding specificity of the ligand of interest and the above protein can be confirmed by a common method using the above substance, which has been labeled or unlabeled.

### 4. Determination of binding ability between protein binding to ligand of interest and control ligand

As a method of determining the binding ability between the protein that has been selected using the binding ability thereof to the ligand of interest as an indicator and a control ligand, a method that is publicly known in the present technical field can be applied.

Examples of such an applicable method include an ELISA method, an RIA method, a surface plasmon resonance (SPR) method, a blotting method, and a method using ligand beads. The control ligand used herein may be labeled. When a substance that specifically binds to a protein specifically binding to the control ligand (for example, a protein such as an antibody, a nucleic acid, a lipid, etc.) can be obtained, the binding specificity of the control ligand and the above protein can be confirmed by a common method using the above substance, which has been labeled or unlabeled.

The degree of determination whether or not it is a protein that binds to a ligand of interest but does not bind to a control ligand is different depending on a method to be applied. For example, when the binding strength is expressed as an O.D. value (absorbance measured at a wavelength suitable for detection of the binding of a protein to a ligand) or the like, if the O.D. value showing the binding ability of the protein to the ligand of interest/the O.D. value showing the binding ability of the protein to the control ligand is at least 2, if such a value is at least 1.0 in the case of using ovalbumin as a control ligand, and if such a value is at least 0.7 in the case of using white-egg lysozyme as a control ligand, it can be determined that it is a protein that specifically binds to the ligand of interest but that does not bind to the control ligand.

It can be determined that the protein obtained via the aforementioned steps is a protein that specifically binds to the ligand of interest.

The examples will be given below. However, these examples are not intended to limit the scope of the present invention.

### EXAMPLES

In the present example, a method, which comprises inducing somatic recombination in immunoglobulin locus and selecting an antibody molecule specifically binding to streptavidin from a DT40 cell population generating various immunoglobulin molecules, will be described. Please refer to WO2004/011644 for a method of preparing such a DT40 cell population.

### 1. Selection of antibody specifically binding to streptavidin

### Cultured cell:

DT40 cells were cultured at 39.5°C in the presence of 5% CO₂ in a 5% CO₂ thermostatic bath. An IMDM medium (Invitrogen) was used as a medium. 10% FBS, 1% chick serum, 100 U/ml penicillin, 100 µg/ml streptomycin, and 55 µM 2-mercaptoethanol were added to the medium, and the obtained medium was used herein. In addition, Trichostatin A (Wako Pure Chemical Industries, Ltd.) was dissolved in methanol, resulting in a concentration of 5 mg/ml, and this mixture was used as a stock. The stock was diluted with a medium as appropriate, resulting in a final concentration of 2.5 ng/ml, and the thus diluted solution was then used. Culture was continued, while keeping a cell concentration of 10⁵ to 10⁶ cells/ml.

### Preparation of streptavidin magnetic beads:

Dynabeads M-280 Tosylactivated (Dynal) was used as magnetic beads, and Dynal MPC (Dynal) was used as a magnetic stand. 200 µl of beads were washed with 500 µl of buffer A (0.1 M sodium phosphate: pH 7.4) three times. Thereafter, the resultant beads were reacted with 240 µg of streptavidin (Nacalai Tesque, Inc.) at 37°C for 24 hours in 400 µl of buffer A, while stirring by rotation. Subsequently, the beads were washed with 500 µl of buffer C (10 mM sodium phosphate: pH 7.4; 150 mM NaCl; and 0.1% BSA) twice. Thereafter, 500 µl of buffer D (0.2 M Tris-HCl: pH 8.5; and 0.1% BSA) was added to the resultant beads, and the obtained mixture was then reacted at 37°C for 4 hours, while stirring by rotation, so as to conduct blocking. Thereafter, the resultant was washed with 500 µl of buffer C twice, and it was then suspended in 400 µl of buffer C that contained 0.02% sodium azide.

### Preparation of rabbit IgG magnetic beads:

Dynabeads M-280 Tosylactivated (Dynal) was used as magnetic beads, and Dynal MPC (Dynal) was used as a magnetic stand. 200 µl of beads were washed with 500 µl of buffer A (0.1 M sodium phosphate: pH 7.4) three times. Thereafter, the resultant beads were reacted with 120 µg of rabbit IgG (SIGMA) at 37°C overnight in 200 µl of buffer A, while stirring by rotation. Subsequently, the beads were washed with 200 µl of buffer C (10 mM sodium phosphate: pH 7.4; 150 mM NaCl; and 0.1% BSA) twice. Thereafter, 200 µl of buffer D (0.2 M Tris-HCl: pH 8.5; and 0.1% BSA) was added to the resultant beads, and the obtained mixture was then reacted at 37°C for 4 hours, while stirring by rotation, so as to conduct blocking. Thereafter, the resultant was washed with 500 µl of buffer C twice, and it was then suspended in 200 µl of buffer C that contained 0.02% sodium azide.

### Selection with streptavidin magnetic beads and rabbit IgG magnetic beads:

Approximately 5 x 10⁷ wild-type DT40 cells, which had been treated with 2.5 ng/ml Trichostatin A for 7 weeks, were washed with 10 ml of a washing buffer (1% BSA-containing PBS) once, and were then washed with 1ml of the washing buffer once. Thereafter, the cells were mixed with 5 x 10⁶ streptavidin magnetic beads (or with rabbit IgG magnetic beads in the case of selection with rabbit IgG magnetic beads) in 1 ml of the washing buffer. Thereafter, the obtained mixture was incubated at 4°C for 30 minutes, while gently rotating it. Thereafter, the resultant was washed with 1 ml of the washing buffer 5 times. Finally, the cells, which had bound to the magnetic beads, were suspended in 500 µl. The obtained suspension was added to 30 ml of the medium, and 300 µl each of the obtained mixture was then dispensed into a 96-well plate, followed by culture at 39.5°C. 1 week later, ELISA was performed on the culture supernatant.

### ELISA using two plates:

Two plates of 96-well Immunoplate Maxisorp (NaigeNunc) were prepared. 200 µl each of 5 µg/ml streptavidin (Nacalai Tesque, Inc.) or ovalbumin (Sigma) (both of which had been dissolved in PBS) was added to either one of the above two plates, and it was then left at room temperature overnight, so as to immobilize them on the plates (rabbit IgG (SIGMA) and white-egg lysozyme were used in the case of selection with the rabbit IgG magnetic beads). Thereafter, blocking was carried out with 200 µl of 0.5% skim milk at room temperature for 1 hour, and the resultant was then washed with 200 µl of an ELISA washing buffer (PBS that contained 0.05% Tween20) 3 times. Thereafter, 100 µl of the cell culture supernatant was added to the resultant, and the obtained mixture was then reacted at room temperature for 1 hour. 100 µl of the same type of culture supernatant was dispensed onto each of the streptavidin-immobilized plate and the ovalbumin-immobilized plate (in the case of selection of rabbit IgG magnetic beads, the rabbit IgG-immobilized plate and the egg-white lysozyme plate). Thereafter, the obtained mixture was washed with 200 µl of an ELISA washing buffer 5 times. Thereafter, a horse radish peroxidase-conjugated goat anti-chick IgM antibody (Bethyl) was diluted with PBS 2000 times, and 100 µl each of the thus diluted antibody was then added to the above resultant. The obtained mixture was reacted at room temperature for 45 minutes, and the reaction product was then washed with the ELISA washing buffer 5 times. Thereafter, 100 µl of TMB+ (Dako) was added to the reaction product, and the obtained mixture was then reacted at room temperature for 4 minutes. Thereafter, 100 µl of 1 N sulfuric acid was added to the reaction system, so as to terminate the reaction. Quantification was carried out by measuring the absorbance at 450 nm, using mQuant Biomolecular Spectrometer (Bio-Tek Instruments).

**[Table 1]**

| **Clone No.** | **Ovalbunin(absorbance)** | **Streptavidin(absorbance)** |
|---|---|---|
| 1 | 0.764 | 0.724 |
| 2 | 2.26 | 1. 611 |
| 3 | 0.543 | 0.47 |
| 4 | 0.185 | 0.155 |
| 5 | 1. 378 | 1. 204 |
| 6 | 0.185 | 0.217 |
| 7 | 0.137 | 0.131 |
| 8 | 0.276 | 0.184 |
| 9 | 0.085 | 0.216 |
| 10 | 0.655 | 0.506 |
| 11 | 0.183 | 0.124 |
| 12 | 0.123 | 0.051 |
| 13 | 0.079 | 0.069 |
| 14 | 0.175 | 0.128 |
| 15 | 0.084 | 0.079 |
| 16 | 0.057 | 0.067 |
| 17 | 0.966 | 0.968 |
| 18 | 0.2 | 0.153 |
| 19 | 0.833 | 0.819 |
| 20 | 0.11 | 0.152 |
| 21 | 0.554 | 0.282 |
| 22 | 0.176 | 1.66 |
| 23 | 0.76 | 0.673 |
| 24 | 0.517 | 0.432 |
| 25 | 2. 883 | 2. 836 |
| 26 | .142 | 0.096 |
| 27 | 0.31 | 0.288 |
| 28 | 0.134 | 0.156 |

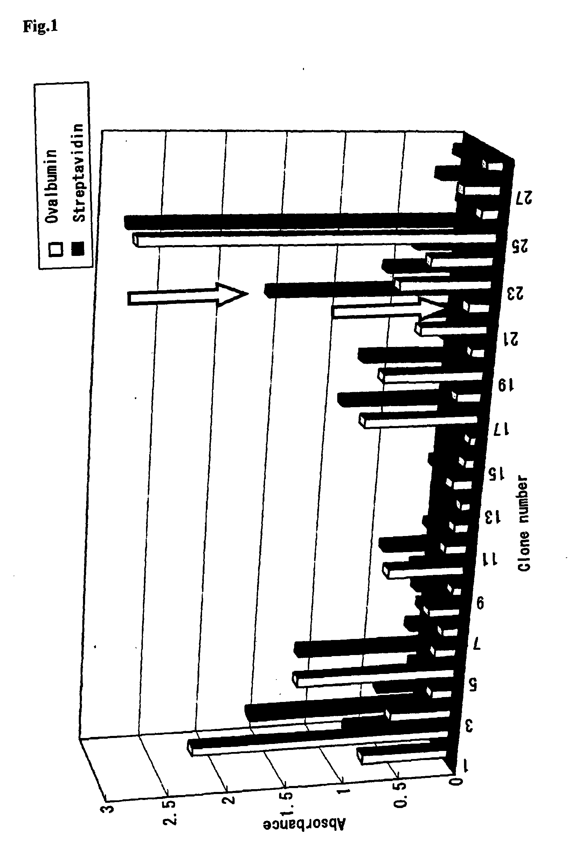

The results of ELISA are shown in Table 1 and Figure 1. Table 1 shows the results of ELISA performed on antibodies reacting with streptavidin (28 clones in total) in the form of O.D. values. Table 1 also shows the results of ELISA regarding the reactivity of the same clones with ovalbumin in the form of O.D. values. The results shown in Table 1 are shown in Figure 1 in the form of a graph.

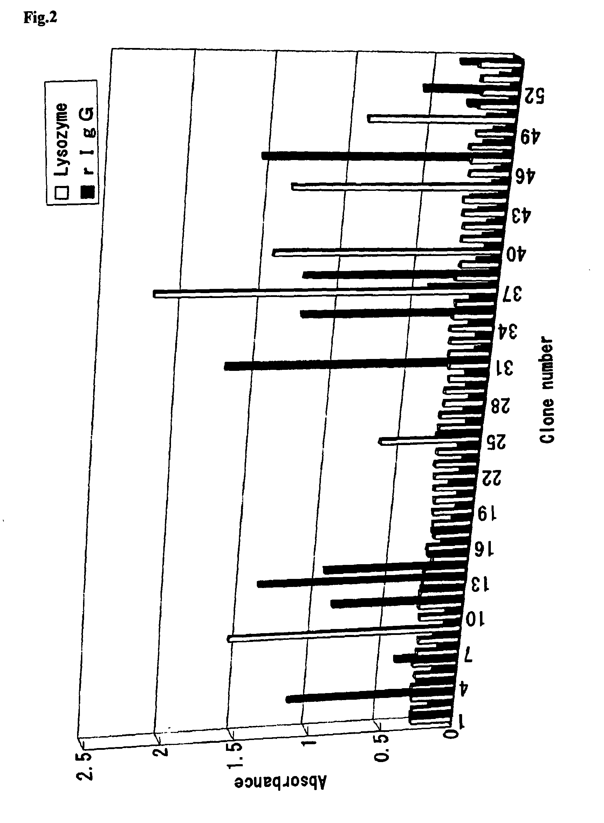

It was revealed that many of the total 28 clones that reacted with streptavidin reacted also with ovalbumin. It was shown that one of these clones (clone 22: named as clone SD-10) specifically reacted only with streptavidin (clone 22 shown in Table 1: refer to the bars indicated with the arrows in Figure 1). All of the clones, which exhibited high affinity for streptavidin, strongly reacted also with ovalbumin. Thus, it was experimentally found that if selection is carried out using only the affinity for streptavidin as an indicator, antibodies that non-specifically bind to any types of ligands are selected with an extremely high probability. On the other hand, it was confirmed that such non-specific antibodies can be efficiently eliminated at the initial stage, using the method of the present invention. Actually, when the assay is carried out by the single use of streptavidin, 28 clones are selected as candidates. Almost of the clones strongly bind to ovalbumin as well. By eliminating such clones, it became possible to immediately determine 1 clone. In the preset experiment, streptavidin was used as a ligand of interest, and ovalbumin was used as a control ligand. However, it was confirmed that this method is effective even in the case of combinations with specific proteins other than the aforementioned proteins (for example, the ligand of interest : the control ligand = rabbit IgG : skim milk, white-egg lysozyme : skim milk). The actual results obtained using white-egg lysozyme are also shown in Figure 2. The presence of cells was observed in 53 wells of the 96-well plate. Out of such 53 clones, 6 clones reacted with rabbit IgG at an O.D. value of 1 or greater (clone Nos. 3, 13, 31, 35, 38, and 47). The reactivity of these clones with lysozyme was simultaneously analyzed. As a result, it could be immediately determined whether or not these 6 clones were specific to rabbit IgG.

### 2. Confirmation of specificity

### Preparation of culture supernatant used in ELISA conducted for studying streptavidin specificity

In order to eliminate IgM derived from serum and the like, a medium as prepared below was used for a culture supernatant that was used to further analyze specificity by ELISA. Immunoglobulin was eliminated in the form of a precipitate from chick serum (Invitrogen), using 50% saturated ammonium sulfate, and the supernatant was then dialyzed to PBS. An increase in the volume generated as a result of the dialysis was corrected by concentration by Centri Prep (Amicon), so as to obtain antibody-eliminated chick serum. The thus obtained serum was added in a concentration of 6% to the AIM-V serum free medium (Invitrogen). Thereafter, cells were added in a concentration of approximately 10⁶/ml to the resultant medium. The obtained mixture was cultured for 2 days, and the culture supernatant was collected, followed by performing ELISA.

### ELISA performed to study streptavidin specificity

200 µl each of 5 µg/ml streptavidin, ovalbumin, human IgG (Sigma), or 0.5% skim milk (Difco) (all of which had been dissolved in PBS) was added to a 96-well Immunoplate Maxisorp, and it was then left at room temperature overnight, so as to immobilize them on the plate. Thereafter, blocking was carried out with 200 µl of 0.5% skim milk at room temperature for 1 hour, and the resultant was then washed with 200 µl of an ELISA washing buffer 3 times. Thereafter, 100 µl of the cell culture supernatant, which had been diluted every 5 times on a scale of 1 to 6, that is, from 1 to 3,125 times, was added to the resultant. The obtained mixture was then reacted at room temperature for 1 hour. Clone SDIO-1 obtained by limiting dilution of the previous SD10 was used as a clone herein. Thereafter, the reaction product was washed with 200 µl of the ELISA washing buffer 5 times. Thereafter, a horse radish peroxidase-conjugated goat anti-chick IgM antibody was diluted with PBS 2000 times, and 100 µl each of the thus diluted antibody was then added to the above resultant. The obtained mixture was reacted at room temperature for 45 minutes, and the reaction product was then washed with the ELISA washing buffer 5 times. Thereafter, 100 µl of TMB+ was added to the reaction product, and the obtained mixture was then reacted at room temperature for 4 minutes. Thereafter, 100 µl of 1 N sulfuric acid was added to the reaction system, so as to terminate the reaction. Quantification was carried out by measuring the absorbance at 450 nm, using mQuant Biomolecular Spectrometer.

The results of ELISA performed to study specificity are shown in Figure 2. It was confirmed that SD10-1 obtained by limiting dilution of SD-10 as shown in Figure 1 strongly reacted with only streptavidin, which had been initially intended. When the present invention is not applied, it is necessary to carry out the same assay on 28 clones. Hence, it was clarified that the present invention has the effect of reducing costs by a factor of at least 28 times.

## Claims

1. A method of selecting from a diversifying library at least one type of protein specifically binding to a ligand of interest, which comprises:
(a) a step of allowing various types of proteins existing in said library to come into contact with the ligand of interest, incubating a mixture consisting of said group of proteins and the ligand of interest, and recovering a complex consisting of at least one type of protein and the ligand of interest;
(b) a step of allowing at least several types of proteins selected in step (a) to come into contact with the ligand of interest, incubating a mixture consisting of said proteins and the ligand of interest, and confirming the binding ability of said proteins to the ligand of interest;
(c) a step of allowing at least several types of proteins selected in step (a) to come into contact with one or two types of specific control ligand(s), incubating a mixture consisting of said proteins and the control ligand(s), and determining whether or not said proteins have bound to the control ligand(s); and
(d) a step of selecting proteins, whose binding ability to the ligand of interest has been confirmed in step (b), and which have not bound to the control ligand(s) in step (c).

2. The method according to claim 1, which is **characterized in that** the proteins that are allowed to come into contact with said ligand of interest in the above step (a) are present on the surface of a host.

3. The method according to claim 1 or 2, which is **characterized in that** said ligand of interest and/or control ligand(s) bind to carriers.

4. The method according to claim 3, which is **characterized in that** said carriers are magnetic beads.

5. The method according to any one of claims 1 to 4, which is **characterized in that** determination of the presence or absence of binding ability in the above step (b) is carried out by a method selected from the group consisting of an ELISA method, an RIA method, a surface plasmon resonance (SPR) method, a blotting method, and a method using ligand beads.

6. The method according to claim 5, which is **characterized in that** said ligand of interest and/or control ligand(s) have been labeled.

7. The method according to any one of claims 1 to 6, which is **characterized in that** said diversifying library is a protein expression library.

8. The method according to claim 7, which is **characterized in that** said protein expression library is an antibody expression library.

9. The method according to claim 8, which is **characterized in that** said antibody expression library is a library that is constituted with cells that present antibodies on the surfaces thereof.

10. The method according to claim 9, which is **characterized in that** said cells are DT40 cells.

11. A protein, which is selected by the method according to any one of claims 1 to 10.
